# EUROPÄISCHE PATENTSCHRIFT

(11) **EP 3 203 952 B1**
(45) Veröffentlichungstag und Bekanntmachung des Hinweises auf die Patenterteilung: **14.04.2021**
(21) Anmeldenummer: 15775226.2
(22) Anmeldetag: 07.10.2015
(51) Int. Cl.: A61F 11/08

(54) **PASSIVER GEHÖRSCHUTZ, DAMIT AUSGESTATTETES OHRPASSSTÜCK UND VERFAHREN ZU DESSEN HERSTELLUNG**
PASSIVE HEARING PROTECTOR, EAR MOULD EQUIPPED THEREWITH AND METHOD FOR THE PRODUCTION THEREOF
DISPOSITIF DE PROTECTION AUDITIVE PASSIVE, ADAPTATEUR AURICULAIRE EQUIPEE D'UN TEL DISPOSITIF ET PROCEDE DE FABRICATION DE CELUI-CI

(30) Priorität: 10.10.2014 DE 102014114736
(43) Veröffentlichungstag der Anmeldung: 16.08.2017
(73) Patentinhaber: bachmaier GmbH & Co. KG, 83486 Ramsau (DE)
(72) Erfinder: KUBICKE, Fabian, 83486 Ramsau (DE)
(74) Vertreter: Winter, Brandl - Partnerschaft mbB
(86) Internationale Anmeldenummer: PCT/EP2015/073192
(87) Internationale Veröffentlichungsnummer: WO 2016/055547

(56) Entgegenhaltungen:
- EP-A1- 0 315 942
- FR-A- 821 677
- US-A- 3 842 829
- US-A1- 2010 329 475

## Beschreibung

Die Erfindung betrifft einen passiven Gehörschutz bzw. ein Passivfilter für den Einsatz in den Gehörgang eines Ohres, gemäß dem Oberbegriff des Anspruchs 1, auf ein mit einem derartigen Gehörschutz ausgestattetes Ohrpassstück und auf ein Verfahren zu dessen Herstellung.

Ein gattungsgemäßer Gehörschutz ist beispielsweise aus dem Dokument EP 0 315 942 B1 bekannt. Außerdem sind Passivfilter dieser Art seit langem auf dem Markt. Entscheidend für die Wirkung und die Leistungsfähigkeit derartiger Passivfilter ist das Zusammenspiel zwischen dem vom Gaze-Netz gebildeten Schall-Dämpfungsglied, der dazu im Abstand gehaltenen biegsamen Membranfolie und dem dahinter liegenden Resonanzvolumen. Da die Abmessungen dieser Passivfilter sehr klein sind - in der Regel haben solche Passivfilter einen Gehäusedurchmesser unter 10 mm, wobei die Dicken der Schall-Dämpfungsglieder und Membranfolien nur bei einigen 1/100-stel mm liegen - kommt es bei der Herstellung derartiger Bauelemente darauf an, die Funktionselemente im Passivfilter reproduzierbar genau zu positionieren und in vorbestimmten Lagezuordnungen zu stabilisieren.

In dem Dokument US 2004/0099473 A1 ist deshalb bereits vorgeschlagen worden, eine als Schall-Dämpfungsglied fungierende Netz- oder Gewebeschicht in spezifischer Weise mit einem Montagematerial zu verbinden, so dass anschließend beim Einkleben des Schall-Dämpfungsglieds in das Filtergehäuse zuverlässig verhindert wird, das Klebstoff auf die Netz- oder Gewebeschicht gelangt. Dieses Verfahren ist aber verhältnismäßig aufwendig. Außerdem hat sich gezeigt, dass es nicht immer gelingt, die Positionierung der Bauteile im Filter exakt einzuhalten.

Der Erfindung liegt deshalb die Aufgabe zugrunde, den gattungsgemäßen Gehörschutz, insbesondere für den Einbau in eine Otoplastik, derart weiterzubilden, dass sich die Herstellung vereinfachen lässt und sich gleichzeitig reproduzierbar eine höhere Genauigkeit bei der Positionierung der Filter-Bauteile zueinander ergibt.

Diese Aufgabe wird durch die Merkmale des Anspruchs 1 gelöst.

Gemäß einem ersten Aspekt bildet ein aus Kunststoff bestehendes Filtergehäuseteil in einem vorbestimmten Axialabstand vom Gaze-Netz eine Innenschulter aus, die in einen von der Innenschulter weg gerichteten kreiszylindrischen Wandabschnitt übergeht, der zur Fixierung der Membranfolie form-, und/oder reib- und/oder materialschlüssig einen sich an der Innenschulter abstützenden Montagering aufnimmt. Der Montagering bildet seinerseits eine radial ausgerichtete Montage-Innenschulter aus, die als ringförmige Auflage für die Membranfolie dient, welche durch einen mit dem Montagering verpressten Haltering, der aus einem im Vergleich zum Montagering härteren Material, vorzugsweise aus Aluminium besteht, flächig gegen die Montage-Innenschulter drückbar ist. Mit dieser Ausgestaltung gelingt es, die Membranfolie ohne den Einsatz von Klebemitteln an einer genau festgelegten Stelle und mit definierten Einspannbedingungen im Gehäuse festzulegen, so dass sich die Lage und Größe des Resonanzvolumens ebenso wie das Schwingungsverhalten der Membranfolie genau festlegen lässt. Dabei ergibt sich der zusätzliche Vorteil, dass der Montageschritt der Membranfolie am Montagering nicht innerhalb des Gehäuses erfolgen muss.

Gemäß der vorteilhaften Weiterbildung nach Anspruch 2 wird der Montagering gleichzeitig dazu herangezogen, das Gaze-Netz in vorbestimmter Lagezuordnung zur Membranfolie im Gehäuse des Gehörschutzes zu fixieren, ohne dass es weiterer Fixierungshilfsmittel, wie Klebstoffe oder dgl. bedarf.

Gemäß einem weiteren Aspekt, der unabhängig vom ersten Aspekt eine eigene Erfindung darstellt, aber auch in Kombination mit dem ersten Aspekt in besonders vorteilhafter Weise zur Lösung der oben angegebenen Aufgabe beiträgt, wird erfindungsgemäß zumindest dasjenige Filtergehäuseteil, welches das Schall-Dämpfungsglied in der Ausgestaltung als Gaze-Netz trägt, als Kunststoff-Spritzgussteil ausgebildet, und das Gaze-Netz wird bei der Herstellung des Filtergehäuseteils randseitig und umlaufend vom Filtergehäuse umspritzt. Durch diese Anordnung wird die Handhabung des extrem dünnen Gaze-Netzes wesentlich vereinfacht, indem MontageHilfsmittel, wie z.B. Klebstoff oder dgl., nicht mehr zu unkontrollierten Verlagerungen des Gaze-Netzes im Gehäuse beitragen können. In Kombination mit dem ersten Aspekt lässt sich auf diese Weise die Relativlage zwischen Gaze-Netz und Membranfolie reproduzierbar auf ein bislang nicht erreichtes, genaues Maß festlegen.

Wenn die Membranfolie von der Innenschulter des Gehäuseteils um ein Maß beabstandet ist, das in der Größenordnung der Dicke des Gaze-Netzes liegt, ergibt sich eine verspannungsarme Einbettung des Gaze-Netzes, mit der die Gefahr von Beschädigungen beim Umspritzen vermindert ist.

Vorteilhafterweise besteht der Haltering - gemäß Anspruch 5 - aus einem im Vergleich zum Montagering härteren Material, vorzugsweise aus Aluminium, wodurch sich beim Eindrücken des Halterings eine zuverlässige Verzahnung mit der Innenwandung des Wandabschnitts herstellen lässt, so dass der Haltering die in der Regel elastische Membranfolie auch nach dem Einpressen zuverlässig in flächiger Anlage gegen die Montage-Innenschulter gedrückt hält.

Vorzugsweise wird der Montagering mit der darin über den Haltering montierten Membranfolie zu einer Montageeinheit zusammengefasst, die unabhängig von den anderen Bestandteilen des Passivfilters herstellbar und als Einheit handhabbar ist.

Der erfindungsgemäße Aufbau des Gehörschutzes bzw. des Passiv-Filters erlaubt es, mit einfachen gießtechnischen Maßnahmen, die Filtercharakteristik zu verändern und an das jeweilige Einsatzgebiet anzupassen. Hierzu ist die Weiterbildung des Anspruchs 6 von besonderem Vorteil, wonach radial außerhalb des kreiszylindrischen Wandabschnitts zumindest ein weiteres Hilfs-Resonanzvolumen ausgebildet wird, welches über eine Durchbruchanordnung vorbestimmter Querschnittsgestaltung, vorzugsweise an der Stirnseite des innenliegenden kreiszylindrischen Wandabschnitts - gemäß Anspruch 8 - mit dem Resonanzvolumen kommuniziert.

Mit einem Aufbau des Filtergehäuses nach Anspruch 9, der unabhängig von der vorstehend beschriebenen Gestaltung des Gehörschutzes als selbständige Erfindung anzusehen ist, lassen sich die Einzelkomponenten des Passiv-Filters mit bislang nicht erreichter Präzision im Filtergehäuse und relativ zueinander dauerhaft positionieren. Weil das Filtergehäuse von zwei über eine umlaufende Zentrierung stirnseitig aneinander gefügten und außenseitig verschweißten, vorzugsweise ultraschallverschweißten Kunststoff-Filtergehäuseteilen gebildet ist, können Montagefehler bei der Verklebung der Gehäuseteile nicht mehr auftreten. Während bislang bei der Verklebung der Gehäuseteile darauf geachtet werden musste, dass der Klebstoff dauerhaft den Kunststoff der Gehäuseteile nicht angreift, werden erfindungsgemäß die vorzugsweise aus demselben Kunststoff bestehenden Gehäuseteile materialschlüssig miteinander verbunden.

Wenn - gemäß Anspruch 10 - das der Schallaustrittsöffnung zugewandte Filtergehäuseteil von einem, mit dem anderen Filtergehäuseteil verschweißbaren plattenförmigen Körper gebildet ist, der einen hohlzylindrischen, hinterschnittenen Montage-Fortsatz für die lösbare Verankerung in einem Ohrpassstück hat, kann das Passiv-Filter kostengünstig für die Ankopplung an verschiedenst gestaltete Ohrpassstücke hergestellt werden. Es ist dann lediglich ein Gehäuseteil mit dem entsprechend gestalteten Montage-Fortsatz auszustatten.

Gute Langzeitstabilität bei prozesssicherer Herstellbarkeit ergibt sich im Besonderen dann, wenn das Filtergehäuse aus einem thermoplastischen Kunststoff aus der Gruppe der synthetischen Polymere und der Familie der Polyester, vorzugsweise aus einem Polycarbonat (PC)-Kunststoff besteht.

Es hat sich gezeigt, dass die Herstellung des Gehörschutzes bzw. des Passiv-Filters dann besonders prozesssicher wird, wenn das Gaze-Netz und/oder die Membranfolie aus einem thermoplastischen Polyester-Kunststoff, vorzugsweise aus Polyethylenterephthalat (PET) gefertigt werden.

Gesonderter Gegenstand des Schutzes ist ein Ohrpassstück gemäß Anspruch 13 mit einem Gehörschutz nach einem der Ansprüche 1 bis 12 und einer zu einer Schalldurchtrittsöffnung offenen hinterschnittenen zylindrischen Ausnehmung, in der ein Montageabschnitt des Gehörschutzes formschlüssig aufgenommen ist.

Besonders wirtschaftliche und prozesssichere Verfahren zur Herstellung eines Gehörschutzes, sind Gegenstand der Ansprüche 14 bis 18.

Mit dem Verfahren nach Anspruch 14 wird die Herstellung des Passiv-Filters weiter vereinfacht, weil eine Montageeinheit mit genau positionierter Membranfolie außerhalb des eigentlichen Filtergehäuses zusammengestellt werden kann.

Der Verfahrensschritt des Anspruchs 15 positioniert den der Montagering mit integrierter Membranfolie zuverlässig und dauerhaft an der Umfangswand des Filtergehäuseteils. Aufgrund des beim Spritzgießen bereits exakt hergestellten Anschlags in Form der Radial-Innenschulter ist die Montage prozesssicher und führt zu einer exakten Positionierung der Membranfolie bezüglich des Gaze-Netzes.

Bei dem Verfahren des Anspruchs 16 wird das in die Spritzgießform eingelegte und darin für den Spritzgießvorgang positionierte Gaze-Netz, welches zuvor aus einer Gaze-Matte ausgestanzt worden ist, nahe einer Radial-Innenschulter mit Kunststoff umspritzt. Dadurch ergibt sich eine bislang unerreichte Positionierungsgenauigkeit des Gaze-Netzes im Gehäuse und relativ zur Innenschulter, was die Voraussetzung für eine genaue Lagebeziehung zwischen Gaze-Netz und Membranfolie schafft.

Die Variante des Verfahrens nach Anspruch 17 vereinfacht das Spritzgießen des Gehäuses, sorgt aber gleichzeitig für eine ausreichend gute Positionierung und Fixierung des Gaze-Netzes im Gehäuse und relativ zur Membranfolie.

Das Verfahren nach Anspruch 18 eliminiert zuverlässig Montagefehler, welche bislang bei der Verklebung der Gehäuseteile auftreten konnten. Weil erfindungsgemäß die vorzugsweise aus demselben Kunststoff bestehenden Gehäuseteile materialschlüssig miteinander verbunden werden, können auch keine Langzeit-Werkstoffveränderungen mehr auftreten, die bislang nur durch eine sorgfältige Anpassung von Gehäusekunststoff und Klebstoff beherrscht werden konnten.

Nachstehend werden anhand schematischer Zeichnungen Ausführungsformen der Erfindung näher beschrieben. Es zeigen:
Figur 1 - stark vergrößert - eine schematische Explosionsdarstellung der Komponenten eines Passiv-Filters für einen erfindungsgemäßen Gehörschutz;
Figur 2 eine der Figur 1 ähnliche Ansicht nach einem Montage-Zwischenschritt;
Figur 3 eine schematische Schnittansicht des Passiv-Filters im fertiggestellten Zustand;
Figur 4 eine schematische - im Vergleich zu den Figuren 1 bis 3 - verkleinerte Darstellung eines Ohrpassstücks mit darin eingesetztem passiven Gehörschutz;
Figur 5 die nochmals vergrößerte Darstellung der Einzelheit "V" in Figur 3;
Figur 6 - stark vergrößert - eine der Figur 1 entsprechende schematische Explosionsdarstellung der Komponenten eines Passiv-Filters für einen erfindungsgemäßen Gehörschutz gemäß einer abgewandelten Ausführungsform;
Figur 7 eine der Figur 6 ähnliche Ansicht nach einem Montage-Zwischenschritt;
Figur 8 eine schematische Schnittansicht des Passiv-Filters gemäß der abgewandelten Ausführungsform im fertiggestellten Zustand; und
Figur 9 die nochmals vergrößerte Darstellung der Einzelheit "IX" in Figur 8.

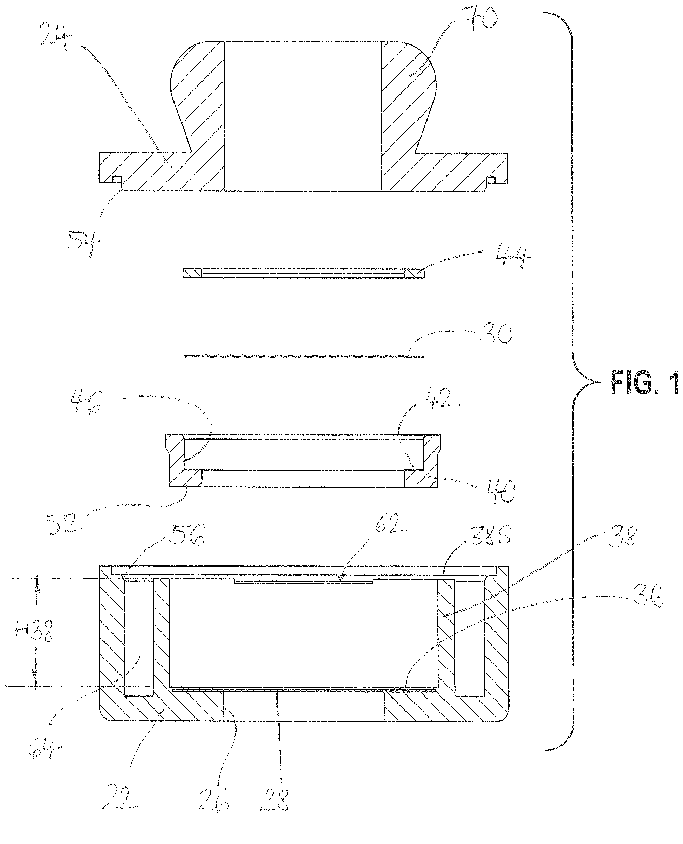

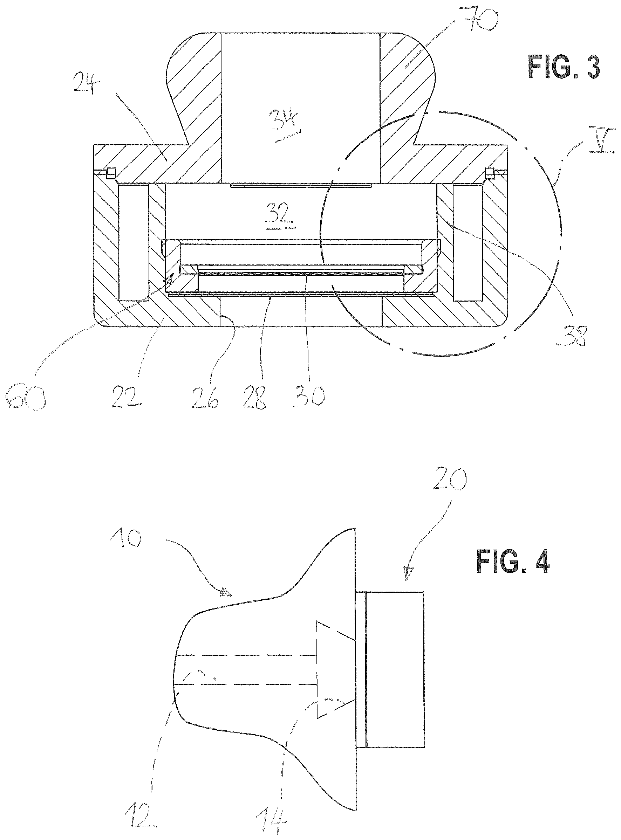

In der Figur 4 ist etwas vergrößert und schematisiert ein der Anatomie des äußeren Ohres angepasstes Ohrpassstück 10 dargestellt, in dem eine Schalldurchtrittsöffnung 12 ausgebildet ist, welche sich zu einer hinterschnittenen zylindrischen Ausnehmung 14 öffnet. Diese zylindrische Ausnehmung 14 dient zur formschlüssigen Aufnahme eines Montageabschnitts 70 eines nachstehend anhand der Figuren 1 bis 3 und 5 näher zu beschreibenden Gehörschutzes in der Ausgestaltung als Passiv-Filter 20, mit dem der in den Gehörgang eintretende Schall selektiv gedämpft werden soll.

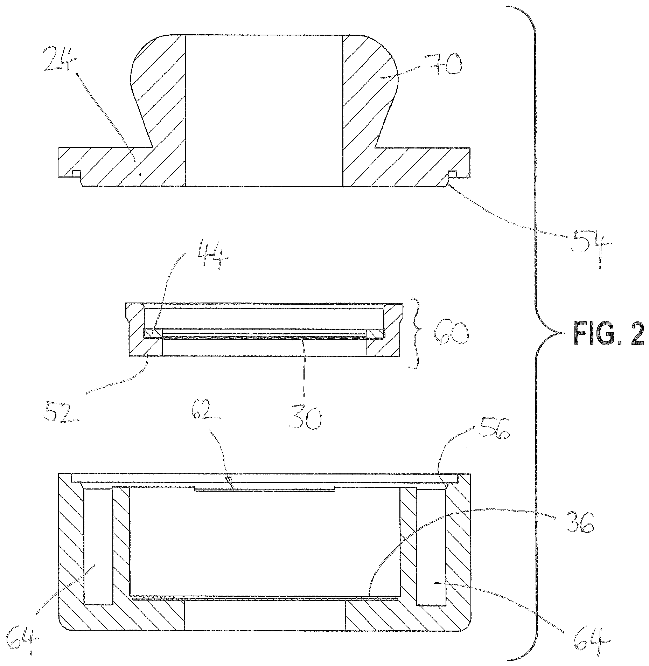

Die Bestandteile des Passiv-Filters 20 sind - in einzelnen frühen Montageschritten - in den Figuren 1 und 2 in stark vergrößertem Maßstab gezeigt. Die Figur 3 zeigt das Passiv-Filter ebenso vergrößert im fertig gestellten Zustand vordem Einsetzen in die Otoplastik. Tatsächlich hat das Gehäuse des Passiv-Filters Abmessungen mit einem Durchmesser von etwa 10 mm und einer axialen Gesamtlänge von etwa 7 mm.

Das Passiv-Filter 20 hat ein rotationssymmetrisches, aus zwei Gehäuseteilen 22 und 24 zusammengesetztes Filtergehäuse, welches vorzugsweise aus einem thermoplastischen Kunststoff aus der Gruppe der synthetischen Polymere und der Familie der Polyester, vorzugsweise aus einem Polycarbonat (PC)-Kunststoff besteht.

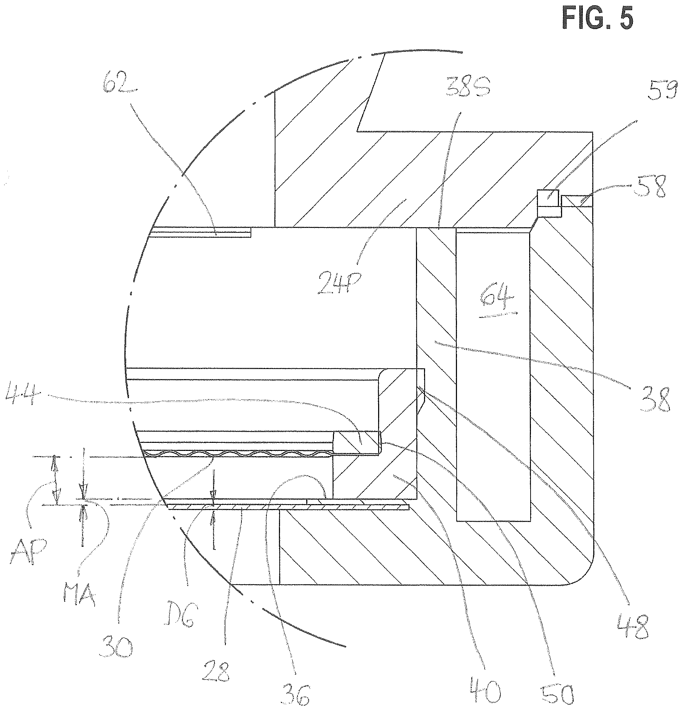

Im Inneren des ersten, d.h. äußeren Filtergehäuseteils 22 ist ortsfest hinter einer Schall-Einlassöffnung 26 ein Gaze-Netz 28 mit vorbestimmter Luftdurchlässigkeit aufgenommen. Das Gaze-Netz 28, dessen Dicke im Bereich von nur einigen 1/100stel mm liegt, ist vorzugsweise aus einem thermoplastischen Polyester-Kunststoff, vorzugsweise aus Polyethylenterephthalat (PET) gefertigt. Die Netzstruktur ist an sich bekannt, so dass hier auf eine genaue Beschreibung verzichtet werden kann.
In einem vorbestimmten Parallelabstand AP - siehe Figur 5 - der in der Größenordnung von nur einem Bruchteil eines mm liegt, nimmt das Gehäuseteil 22 Membranfolie 30 auf, hinter dem sich ein Resonanzvolumen 32 befindet. Das Resonanzvolumen 32 kommuniziert mit einer Schallaustrittsöffnung 34, die sich im Inneren des Filtergehäuseteils 24, genauer gesagt im Inneren des Montageabschnitts 70 befindet.

Die Membranfolie 30, welche in der Regel ebenfalls eine Stärke von nur einigen 1/100stel mm hat, ist vorzugsweise ebenfalls aus einem thermoplastischen Polyester-Kunststoff, vorzugsweise aus Polyethylenterephthalat (PET) gefertigt. Derartige Membranfolien sind an sich ebenfalls bekannt, so dass auf die Struktur hier nicht näher eingegangen werden soll.

Die Besonderheit des Passiv-Filters ist im besonderen Aufbau zu sehen, der ein verbessertes Herstellungsverfahren eröffnet, welches nachstehend näher erläutert werden soll.

Wie aus den Figuren 1 bis 3 und 5 ersichtlich bildet das Filtergehäuseteil 22 hinter der Schall-Einlassöffnung 26 eine Innenschulter 36 aus, die in einen von der Innenschulter 36 weg gerichteten kreiszylindrischen Wandabschnitt 38 übergeht. Der Wandabschnitt 38 dient zur Fixierung der Membranfolie 30 wie folgt: am Wandabschnitt 38 ist form-, und/oder reib- und/oder materialschlüssig ein sich an der Innenschulter 36 abstützender Montagering 40 aufgenommen, welcher seinerseits eine radial ausgerichtete Montage-Innenschulter 42 ausbildet. Die Montage-Innenschulter 42 dient als ringförmige Auflage für die durch einen eingepressten Haltering 44 fixierte Membranfolie 30.

Der Haltering 44 besteht aus einem im Vergleich zum Montagering 40, der aus einem thermoplastischen Kunststoff aus der Gruppe der synthetischen Polymere und der Familie der Polyester, vorzugsweise aus einem Polycarbonat (PC)-Kunststoff gebildet sein kann, härteren Material, vorzugsweise aus Aluminium, und er drückt die Membranfolie 30 beim Einpressen in die kreiszylindrische Innenausnehmung 46 flächig gegen die Montage-Innenschulter 42. Über eine in Figur 5 angedeutete Verzahnung 50 verkeilt sich der Haltering 44 fest und dauerhaft mit der kreiszylindrischen Innenausnehmung 46 und positioniert so die in ein bestimmtes Relief geprägte Membranfolie 30 in einem exakt festgelegten Abstand zur Auflagefläche 52 des Montage rings 40. Der Montagering 40 mit der darin über den Haltering 44 montierten Membranfolie 30 lässt sich auf diese Weise zu einer Montageeinheit 60 zusammenfassen, was in Figur 2 dargestellt ist. Durch Einpressen der Montageeinheit 60 in den Wandabschnitt 38 auf Anschlag mit der Innenschulter 36 ist die Membranfolie 30 innerhalb des Gehäuses 22 exakt fixiert, ohne dass es irgendwelcher weiteren Befestigungsmittel, wie z.B. Klebstoffs, bedarf. Eine mit 48 bezeichnete Rändelung oder Verzahnung sorgt für einen sicheren Halt der Montageeinheit 60.

Damit der vorbestimmte Parallelabstand AP - siehe Figur 5 - zwischen Gaze-Netz 28 Membranfolie 30 zuverlässig und möglichst prozesssicher bzw. reproduzierbar eingehalten wird, ist das Filtergehäuseteil 22, welches das Gaze-Netz 28 trägt, als Kunststoff-Spritzgussteil ausgebildet ist, und das Gaze-Netz 28 ist randseitig und umlaufend vom Filtergehäuse 22 umspritzt, was am besten aus der Figur 5 erkennbar ist. Dabei liegt das Gaze-Netz 28 von der Innenschulter 36 vorzugsweise um ein Maß MA beabstandet, das in der Größenordnung der Dicke DG des Gaze-Netzes 28 liegt. Dies lässt sich beispielsweise dadurch erreichen, dass ein zuvor aus einer Gaze-Matte ausgestanztes Gaze-Netz 28 in die Spritzgießform eingelegt und darin durch geeignete, beispielsweise verlorene Kerne während des Umspritzvorgangs positioniert wird.

Zur Fertigstellung des Passiv-Filters 20 mit den beiden Filtergehäuseteilen 22 und 24 mit darin bereits fertig positioniertem Gaze-Netz 28 und Membranfolie 30, werden die Filtergehäuseteile 22 und 24 zentriert - über angedeutete umlaufende Zentrierschrägen 54, 56 - stirnseitig aneinander gefügt und außenseitig verschweißt, vorzugsweise ultraschallverschweißt. Zu diesem Zweck sind im Bereich der Fügestellen Verzahnungsbereiche 58 und Freistiche 59 vorgesehen, damit beim Verschweißen auftretendes geschmolzenes Kunststoffmaterial in der Fügestelle aufgenommen werden kann, ohne die Positionierung der Gehäuseteile zueinander zu beeinträchtigen.

Man erkennt aus der Darstellung in den Figuren, dass das die Schallaustrittsöffnung 34 ausbildende Filtergehäuseteil 24 von einem mit dem anderen Filtergehäuseteil 22 verschweißbaren plattenförmigen Körper gebildet ist, der einen hohlzylindrischen, hinterschnittenen Montage-Fortsatz 70 für die lösbare Verankerung im Ohrpassstück 10 hat. Gleichzeitig wird die plattenartige Ausbildung des Filtergehäuseteils 24 dazu herangezogen, das Resonanzverhalten des Passiv-Filters zu steuern, und zwar wie folgt:
Wenn - wie in Figur 3 und 5 gezeigt - die beiden Gehäuseteile 22, 24 aufeinander gesetzt und miteinander verbunden werden, legt sich der plattenartige Gehäuseabschnitt 24P plan an die Stirnseite 38S des Wandabschnitts 38, wodurch das Resonanzvolumen 32 abgeschlossen wird. Der kreiszylindrische Wandabschnitt 38 hat bei der gezeigten Ausführungsform zumindest eine, vorzugsweise mehrere im Umfangsabstand zueinander stehende stirnseitige Aussparungen 62 - siehe Figur 5 - über die das Resonanzvolumen 32 mit zumindest einem weiteres Hilfs-Resonanzvolumen 64, welches sich radial außerhalb des kreiszylindrischen Wandabschnitts 38 befindet, kommunizieren kann. Über die Geometrie der Durchbruchanordnung in Form der stirnseitigen Aussparungen 62 kann auf die Filtercharakteristik mit einfachen Mitteln Einfluss genommen werden. Das Hilfs-Resonanzvolumen 64 kann - gemäß einer nicht näher dargestellten Ausführungsform - auch gänzlich entfallen.

Bei der Herstellung des passiven Gehörschutzes geht man vorzugsweise wie folgt vor:
Zunächst wird im Spritzgussverfahren ein rotationssymmetrisches Bodenteil 22 mit einer bodenseitigen Eintrittsöffnung 26 und dahinter liegender Radial-Innenschulter 36 und einer gegebenenfalls doppelwandigen Umfangswand 38 vorbestimmter Höhe H38 hergestellt, wobei ein in die Spritzgießform eingelegtes und darin positioniertes Gaze-Netz 28, welches zuvor aus einer Gaze-Matte ausgestanzt worden ist, nahe der Radial-Innenschulter 36, also in einem Abstand MA umspritzt wird.

Dann wird ein Montagering 40 mit einer radial ausgerichteten Montage-Innenschulter 42 zur Ausbildung einer ringförmigen Auflage für eine Membranfolie 30 mit einem Haltering 44, der vorzugsweise aus Aluminium besteht, zu einer Montageeinheit 60 zusammengefasst, indem der Haltering 44 in eine kreiszylindrische Innenausnehmung 46 des Montagerings 40 mit Kraft- und/oder Formschluss eingepresst und dabei die Membranfolie 30 flächig gegen die Montage-Innenschulter 42 gedrückt wird.

Dann wird der Montagering 40 mit integrierter Membranfolie 30 mit Kraft- und/oder Formschluss in die Umfangswand 38 des Filtergehäuseteils 22 auf unmittelbaren Anschlag mit der Radial-Innenschulter 36 gepresst.

Schließlich wird auf das Filtergehäuseteil 22 mit darin verankerter Montageeinheit 60 mit Montagering 40, Membranfolie 30 und Haltering 44 mit umlaufender Zentrierung 54, 56 ein das Resonanzvolumen 32 abschließendes Deckelgehäuseteil 24 aufgesetzt, woraufhin die beiden Gehäuseteile 22, 24 außenseitig und umlaufend verschweißt, vorzugsweise ultraschallverschweißt, werden.

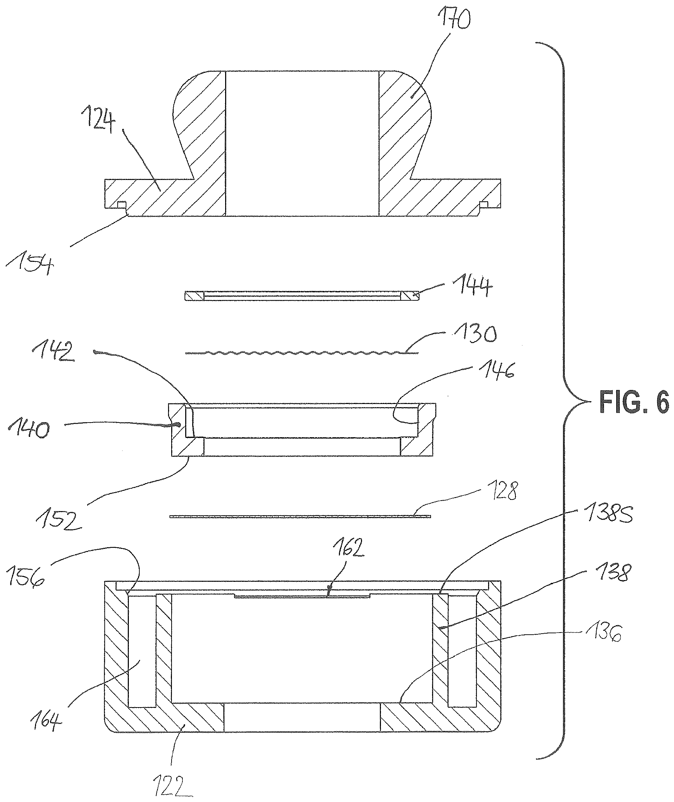

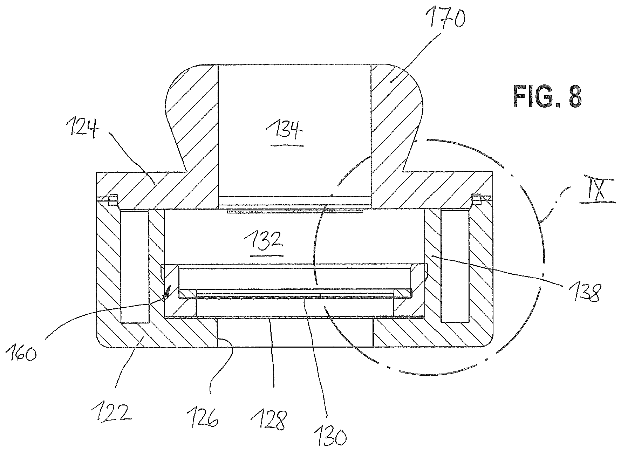

Nachstehend wird anhand der Figuren 6 bis 9 eine weitere Ausführungsform der Erfindung beschrieben. Die Darstellungen in den Figuren 6 bis 9 entsprechen hinsichtlich Maßstab und Aufbau den Figuren 1 bis 3 und 5. Außerdem sind diejenigen Bauelemente, die den Komponenten der Ausführungsform der Figuren 1 bis 5 entsprechen, mit gleichen Bezugszeichen versehen, denen allerdings eine "1" vorangestellt wurde.

Der alleinige Unterschied zwischen der Ausführungsform nach den Figuren 1 bis 5 und der Ausführungsform nach Figur 6 bis 9 besteht in der Positionierung und Fixierung des Gaze-Netzes 128 im Gehäuseteil 122.

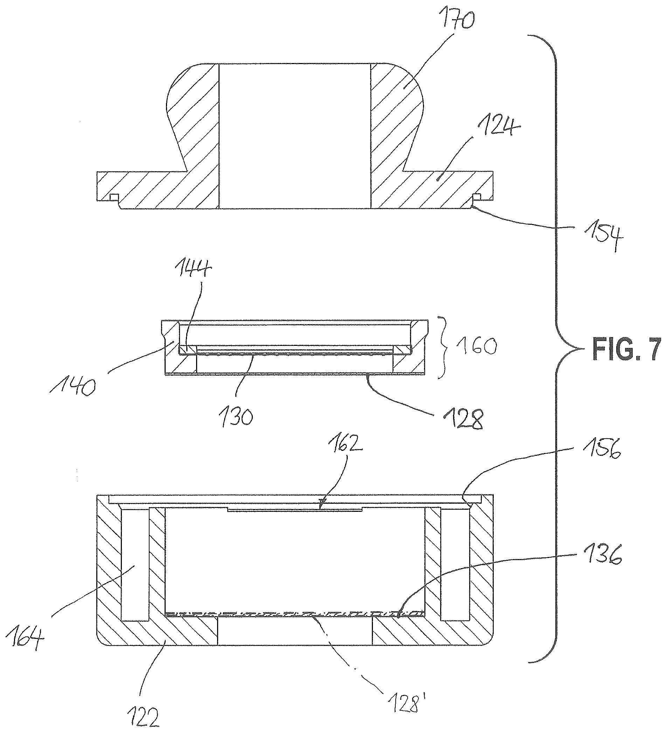

Wie aus Figur 6 ersichtlich stellt das Gaze-Netz 128, welches wieder aus einer Gaze-Matte formgenau ausgestanzt sein kann, ein vom Gehäuseteil 122 getrenntes Bauelement dar, welches vorzugsweise mit Führung über seinen Umfang in das Gehäuseteil 122 derart einsetzbar ist, dass es flächig auf der Radial-Innenschulter 136 aufliegt. Für diesen Montagevorgang kann das Stanzwerkzeug herangezogen werden, wobei das Einlegen durch einen geeignet gestalteten Stempel unterstützt wird. Der im Gehäuse 122 eigelegte Zustand des Gaze-Netzes 128' ist in Figur 7 mit strichpunktierter Linie dargestellt.

Es ist jedoch - gemäß einer Variante der Erfindung - auch möglich, die aus Montagering 140, Membranfolie 130 und Haltering 144 zusammengestellte Montageeinheit 160 um das Gaze-Netz 128 zu erweitern - wie in Figur 7 mit durchgezogener Linie dargestellt - und diese Montageeinheit dann wie im Zusammenhang mit der Ausführungsform gemäß Figuren 1 bis 5 beschrieben in das Gehäuseteil 122 auf Anschlag mit der Innenschulter 136 einzupressen.

Der Montagering 140 schlägt in diesem Fall also mittelbar, d.h. über das Gaze-Netz 128 bzw. 128', an der Radial-Innenschulter 136 an. Es hat sich gezeigt, dass das Gaze-Netz 128 beim Montagevorgang durch geringste Adhäsionskräfte an der Unterseite des Montagerings 140 gehalten werden kann, ohne dass ein Klebstoff oder andere Zusatzmittel erforderlich sind.

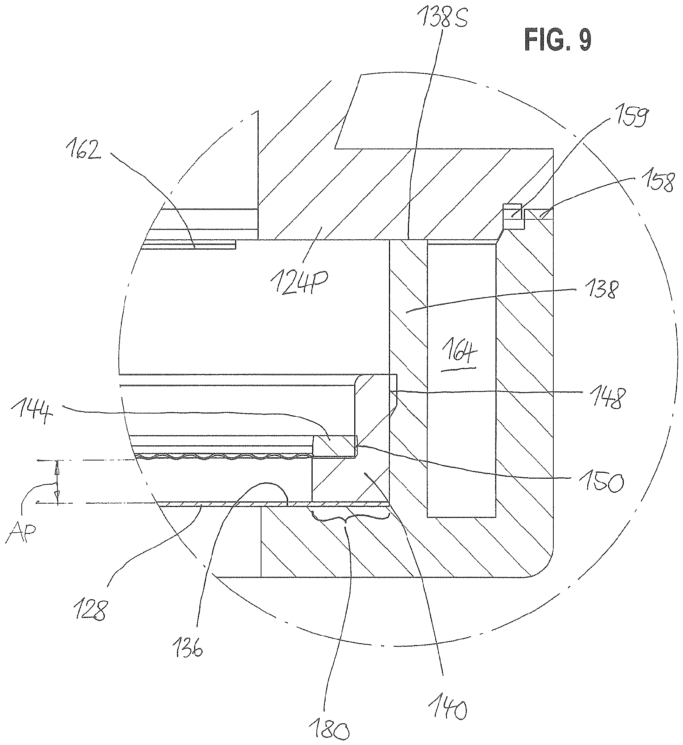

Der in das Gehäuse 122 eingepresste Montagering 140 positioniert somit das Gaze-Netz 128 bzw. 128' - wie in Figur 9 in Einzelheit gezeigt - auf der Innenschulter 136 über einen ausreichend großen Ringflächenbereich 180, um sicherzustellen, dass das Gaze-Netz 128 über die gesamte Lebensdauer des Passiv-Filters zuverlässig in exakter Position auch unter Einhaltung des Abstandes AP gehalten wird.

Die übrigen Funktionselemente der Ausführungsform gemäß Figuren 6 bis 9 sind mit denjenigen der Ausführungsform gemäß Figur 1 bis 5 identisch, so dass auf eine erneute Beschreibung dieser Teile verzichtet werden kann. Auch die Verfahrensschritte bei der Herstellung des Gehörschutzes nach Figur 6 bis 9 sind - mit Ausnahme der fehlenden Umspritzung und der deshalb modifizierten Fixierung des Gaze-Netzes 128 im Gehäuse 122 - mit den Herstellungsschritten der zuvor beschriebenen Ausführungsform vergleichbar.

Die Erfindung schafft somit einen Gehörschutz für den Einsatz in den Gehörgang eines Ohres, insbesondere für den Einsatz in ein Ohrpassstück, mit einem rotationssymmetrischen, aus mehreren Gehäuseteilen 22, 24 zusammengesetzten Filtergehäuse, welches in seinem Inneren ortsfest ein hinter einer Schall-Einlassöffnung 26 liegendes Gaze-Netz 28 mit vorbestimmter Luftdurchlässigkeit und im Parallelabstand AP dazu eine Membranfolie 30 aufnimmt, hinter dem sich ein Resonanzvolumen 32 befindet, welches mit einer Schallaustrittsöffnung 34 kommuniziert. Zur Verbesserung der Genauigkeit der Lageanordnung der Funktionskomponenten im Gehäuse des Gehörschutzes bildet ein Filtergehäuseteil hinter der Schall-Einlassöffnung 26 eine Innenschulter 36 aus, die in einen von ihr weg gerichteten kreiszylindrischen Wandabschnitt 38 übergeht. Der Wandabschnitt nimmt zur Fixierung der Membranfolie 30 form-, und/oder reib- und/oder materialschlüssig einen sich an der Innenschulter 36 abstützenden Montagering 40 auf, welcher seinerseits eine radial ausgerichtete Montage-Innenschulter 42 als ringförmige Auflage für die durch einen eingepressten Haltering 44 fixierte Membranfolie 30 ausbildet. Der Montagering kann gleichzeitig zur Fixierung des Gaze-Netzes herangezogen werden. Alternativ oder in Kombination mit der Ausgestaltung des Montagerings ist zumindest dasjenige Filtergehäuseteil 22, welches das Gaze-Netz 28 trägt, als Kunststoff-Spritzgussteil ausgebildet ist, und das Gaze-Netz 28 ist randseitig und umlaufend vom Filtergehäuse 22 umspritzt.

## Patentansprüche

1. Passiver Gehörschutz für den Einsatz in den Gehörgang eines Ohres, insbesondere für den Einsatz in ein Ohrpassstück, mit einem rotationssymmetrischen, aus mehreren Gehäuseteilen (22, 24; 122, 124) zusammengesetzten Filtergehäuse, welches in seinem Inneren ortsfest ein hinter einer Schall-Einlassöffnung (26; 126) liegendes Gaze-Netz (28; 128) mit vorbestimmter Luftdurchlässigkeit und im Parallelabstand (AP) dazu eine Membranfolie (30; 130) aufnimmt, hinter dem sich ein Resonanzvolumen (32; 132) befindet, welches mit einer Schallaustrittsöffnung (34; 134) kommuniziert, **dadurch gekennzeichnet, dass** ein Filtergehäuseteil (22; 122) hinter der Schall-Einlassöffnung (26; 126) eine Innenschulter (36; 136) ausbildet, die in einen von der Innenschulter (36; 136) weg gerichteten kreiszylindrischen Wandabschnitt (38; 138) übergeht, der zur Fixierung der Membranfolie (30; 130) form-, und/oder reib- und/oder materialschlüssig einen sich an der Innenschulter (36; 136) abstützenden Montagering (40; 140) aufnimmt, welcher seinerseits eine radial ausgerichtete Montage-Innenschulter (42; 142) als ringförmige Auflage für die durch einen eingepressten Haltering (44; 144) fixierte Membranfolie (30; 130) ausbildet.

2. Gehörschutz nach Anspruch 1, **dadurch gekennzeichnet, dass** der Montagering (140) das Gaze-Netz (128) gegen die Innenschulter (136) drückt und dadurch im Gehäuse (122) positioniert.

3. Gehörschutz, insbesondere nach Anspruch 1, mit einem rotationssymmetrischen, aus mehreren Gehäuseteilen (22, 24) zusammengesetzten Filtergehäuse, welches in seinem Inneren ortsfest ein hinter einer Schall-Einlassöffnung (26) liegendes Gaze-Netz (28) mit vorbestimmter Luftdurchlässigkeit und im Parallelabstand (AP) dazu eine Membranfolie (30) aufnimmt, hinter dem sich ein Resonanzvolumen (32) befindet, welches mit einer Schallaustrittsöffnung (34) kommuniziert, **dadurch gekennzeichnet, dass** zumindest dasjenige Filtergehäuseteil (22), welches das Gaze-Netz (28) trägt, als Kunststoff-Spritzgussteil ausgebildet ist, und das Gaze-Netz (28) randseitig und umlaufend vom Filtergehäuse (22) umspritzt ist.

4. Gehörschutz nach Anspruch 3, **dadurch gekennzeichnet, dass** das Gaze-Netz (28) von der Innenschulter (36) um ein Maß (MA) beabstandet ist, das in der Größenordnung der Dicke (DG) des Gaze-Netzes (28) liegt.

5. Gehörschutz nach einem der Ansprüche 1 bis 4, **dadurch gekennzeichnet, dass** der Haltering (44; 144) aus einem im Vergleich zum Montagering (40; 140) härteren Material, vorzugsweise aus Aluminium, besteht und die Membranfolie (30; 130) flächig gegen die Montage-Innenschulter (42; 142) drückt.

6. Gehörschutz nach Anspruch 5, **dadurch gekennzeichnet, dass** der Montagering (40; 140) mit darin über den Haltering (44; 144) montierter Membranfolie (30; 130) als Montageeinheit (60; 160) zusammengefasst ist.

7. Gehörschutz nach einem der Ansprüche 1 bis 6, **dadurch gekennzeichnet, dass** radial außerhalb des kreiszylindrischen Wandabschnitts (38; 138) zumindest ein weiteres Hilfs-Resonanzvolumen (64; 164) ausgebildet ist, welches über eine Durchbruchanordnung (62; 162) vorbestimmter Querschnittsgestaltung mit dem Resonanzvolumen (32; 132) kommuniziert.

8. Gehörschutz nach Anspruch 7, **dadurch gekennzeichnet, dass** das Hilfs-Resonanzvolumen (64; 164) von einer ringförmig umlaufenden Resonanzkammer (64; 164) gebildet ist, die über stirnseitige Aussparungen (62; 162) des kreiszylindrischen Wandabschnitts (38; 138) mit dem Resonanzvolumen (32; 132) kommuniziert.

9. Gehörschutz insbesondere nach einem der Ansprüche 1 bis 8, mit einem rotationssymmetrischen, aus mehreren Gehäuseteilen (22, 24; 122, 124) zusammengesetzten Filtergehäuse, welches in seinem Inneren ortsfest ein hinter einer Schall-Einlassöffnung (26; 126) liegendes Gaze-Netz (28; 128) mit vorbestimmter Luftdurchlässigkeit und im Parallelabstand (AP) dazu eine Membranfolie (30); 130 aufnimmt, hinter dem sich ein Resonanzvolumen (32; 132) befindet, welches mit einer Schallaustrittsöffnung (34; 134) kommuniziert, **dadurch gekennzeichnet, dass** das Filtergehäuse (22, 24; 122, 124) von zwei über eine umlaufende Zentrierung (54, 56; 154, 165) stirnseitig aneinander gefügten und außenseitig verschweißten, vorzugsweise ultraschallverschweißten Kunststoff-Filtergehäuseteilen (22, 24; 122, 124) gebildet ist.

10. Gehörschutz nach Anspruch 9, **dadurch gekennzeichnet, dass** das der Schallaustrittsöffnung (34; 134) zugewandte Filtergehäuseteil (24; 124) von einem, mit dem anderen Filtergehäuseteil (22; 122) verschweißbaren plattenförmigen Körper (24P; 124P) gebildet ist, der einen hohlzylindrischen, hinterschnittenen Montage-Fortsatz (70; 170) für die lösbare Verankerung in einem Ohrpassstück (10) hat.

11. Gehörschutz nach einem der Ansprüche 1 bis 10, **dadurch gekennzeichnet, dass** das Filtergehäuse (22, 24; 122, 124) aus einem thermoplastischen Kunststoff aus der Gruppe der synthetischen Polymere und der Familie der Polyester, vorzugsweise aus einem Polycarbonat (PC)-Kunststoff besteht.

12. Gehörschutz nach einem der Ansprüche 1 bis 11, **dadurch gekennzeichnet, dass** das Gaze-Netz (28; 128) und/oder die Membranfolie (30; 130) aus einem thermoplastischen Polyester-Kunststoff, vorzugsweise aus Polyethylenterephthalat (PET) gefertigt ist.

13. Ohrpassstück mit einem Gehörschutz nach einem der Ansprüche 1 bis 12, wobei der Gehörschutz einen Montage-Fortsatz (70; 170) aufweist und das Ohrpassstück eine zu einer Schalldurchtrittsöffnung (12) offene hinterschnittene zylindrische Ausnehmung (14) für die formschlüssige Aufnahme des Montage-Fortsatzes (70; 170) aufweist.

14. Verfahren zur Herstellung eines Gehörschutzes nach einem der Ansprüche 1 bis 13, **dadurch gekennzeichnet, dass** ein erstes Filtergehäuseteil (22; 122) im Spritzgussverfahren in der Weise hergestellt wird, dass ein rotationssymmetrisches Bodenteil mit einer bodenseitigen Eintrittsöffnung (26; 126) und dahinter liegender Radial-Innenschulter (36; 136) und einer gegebenenfalls doppelwandigen Umfangswand (38; 138) vorbestimmter Höhe (H38) entsteht, wobei ein Montagering (40; 140) mit einer radial ausgerichteten Montage-Innenschulter (42; 142) zur Ausbildung einer ringförmigen Auflage für eine Membranfolie (30; 130) mit einem in eine kreiszylindrische Innenausnehmung (46; 146) mit Kraft- und/oder Formschluss eingepressten und die Membranfolie (30; 130) flächig gegen die Montage-Innenschulter (42; 142) drückenden Haltering (44; 144), der vorzugsweise aus Aluminium besteht, zu einer Montageeinheit (60; 160) zusammengefasst wird.

15. Verfahren nach Anspruch 14, **dadurch gekennzeichnet, dass** der Montagering (40; 140) mit integrierter Membranfolie (30; 130) mit Kraft- und/oder Formschluss in die Umfangswand (38; 138) des Filtergehäuseteils (22; 122) auf mittelbaren oder unmittelbaren Anschlag mit der Radial-Innenschulter (36; 136) gepresst wird.

16. Verfahren nach Anspruch 15, **dadurch gekennzeichnet, dass** ein in die Spritzgießform eingelegtes und darin positioniertes Gaze-Netz (28), welches zuvor aus einer Gaze-Matte ausgestanzt worden ist, nahe der Radial-Innenschulter (36) umspritzt wird, und der Montagering (40) auf unmittelbaren Anschlag mit der Radial-Innenschulter (36) gepresst wird.

17. Verfahren nach Anspruch 15, **dadurch gekennzeichnet, dass** die Montageeinheit (160) ein Gaze-Netz (128), welches aus einer Gaze-Matte passgenau ausgestanzt und vorzugsweise umfangsseitig geführt auf die Radial-Innenschulter (136) gelegt worden ist, gegen die Radial-Innenschulter (136) presst und damit im Gehäuse (122) fixiert.

18. Verfahren insbesondere nach einem der Ansprüche 14bis 17, **dadurch gekennzeichnet** das auf ein erstes Filtergehäuseteil (22; 122) mit darin verankerter Montageeinheit (60; 160) mit Montagering (40; 140), Membranfolie (30; 130) und Haltering (44; 144) mit umlaufender Zentrierung (54, 56; 154, 156) ein ein Resonanzvolumen (32; 132) abschließendes Deckelgehäuseteil (24; 124) aufgesetzt und außenseitig umlaufend mit dem ersten Filtergehäuseteil (22; 122) verschweißt, vorzugsweise ultraschallverschweißt, wird.

## Claims

1. Passive hearing protector for use in the auditory canal of an ear, in particular for use in an ear mould, comprising a rotationally symmetrical filter housing composed of a plurality of housing parts (22, 24; 122, 124), which filter housing has in its interior at a fixed position a gauze net (28; 128) with a predetermined air permeability being provided behind a sound inlet opening (26; 126) and at a parallel distance (AP) thereto a membrane film (30; 130), behind which filter housing a resonance volume (32; 132) communicating with a sound outlet opening (34; 134) is provided,
**characterized in that**
a filter housing part (22; 122) behind the sound inlet opening (26; 126) forms an inner shoulder (36; 136), which merges into a circular-cylindrical wall section (38; 138) directed away from the inner shoulder (36; 136), the wall section (38; 138) forming a mounting ring (40; 140) supported on the inner shoulder (36; 136) in a form-fitting and/or friction-fitting and/or material-fitting manner for fixation of the membrane film (30; 130), the mounting ring (40; 140) itself forming a radially directed mounting inner shoulder (42; 142) as an annular support for the membrane film (30; 130) being fixed by a pressed-in retaining ring (44; 144).

2. Hearing protector according to claim 1, **characterized in that** the mounting ring (140) presses the gauze net (128) against the inner shoulder (136) and thereby positions it in the housing (122).

3. Hearing protector, in particular according to claim 1, comprising a rotationally symmetrical filter housing composed of a plurality of housing parts (22, 24), which filter housing has in its interior at a fixed position a gauze net (28) with a predetermined air permeability being provided behind a sound inlet opening (26) and a membrane film (30) at a parallel distance (AP) thereto, behind which filter housing a resonance volume (32) communicating with a sound outlet opening (34) is provided, **characterized in that** at least said filter housing part (22) which holds the gauze net (28) is formed as a plastic injection-moulded part, and **in that** the gauze net (28) is over-moulded at the rim-side thereof and circumferentially by the filter housing (22).

4. Hearing protector according to claim 3, **characterized in that** the gauze net (28) is spaced apart from the inner shoulder (36) by an amount (MA) which is in the order of magnitude of the thickness (DG) of the gauze net (28).

5. Hearing protector according to one of claims 1 to 4, **characterized in that** the retaining ring (44; 144) consists of a material harder than the mounting ring (40; 140), preferably of aluminum, and **in that** the membrane film (30; 130) presses flatly against the mounting inner shoulder (42; 142).

6. Hearing protector according to claim 5, **characterized in that** the mounting ring (40; 140) is combined with the membrane film (30; 130) mounted therein via the retaining ring (44; 144) as a mounting unit (60; 160).

7. Hearing protector according to one of claims 1 to 6, **characterized in that** radially outside of the circular-cylindrical wall section (38; 138) at least one further auxiliary resonance volume (64; 164) is formed, which communicates with the resonance volume (32; 132) via an opening arrangement (62; 162) of predetermined cross-sectional design.

8. Hearing protector according to claim 7, **characterized in that** the auxiliary resonance volume (64; 164) is formed by an annularly encircling resonance chamber (64; 164) which communicates with the resonance volume (32; 132) via face-side recesses (62; 162) of the circular-cylindrical wall section (38; 138).

9. Hearing protector in particular according to one of claims 1 to 8, comprising a rotationally symmetrical filter housing composed of a plurality of housing parts (22, 24; 122, 124), which filter housing has in its interior at a fixed position a gauze net (28; 128) with a predetermined air permeability being provided behind a sound inlet opening (26; 126) and at a parallel distance (AP) thereto a membrane film (30; 130), behind which filter housing a resonance volume (32; 132) communicating with a sound outlet opening (34; 134) is provided, **characterized in that** the filter housing (22, 24; 122, 124) is formed by two plastic filter housing parts (22, 24; 122, 124) being joined to one another at their end faces by means of a circumferential centering (54, 56; 154, 165) and being welded on the outside, preferably ultrasonically welded.

10. Hearing protector according to claim 9, **characterized in that** the one filter housing part (24; 124) facing the sound outlet opening (34; 134) is formed by a plate-shaped body (24P; 124P) being weldable to the other filter housing part (22; 122) which has a hollow-cylindrical, undercut mounting extension (70; 170) for detachable anchoring in an ear mould (10).

11. Hearing protector according to one of claims 1 to 10, **characterized in that** the filter housing (22, 24; 122, 124) consists of a thermoplastic material from the group of synthetic polymers and the family of polyesters, preferably of a polycarbonate (PC) plastic.

12. Hearing protector according to one of claims 1 to 11, **characterized in that** the gauze net (28; 128) and/or the membrane film (30; 130) is made of a thermoplastic polyester plastic, preferably of polyethylene terephthalate (PET).

13. Ear mould comprising a hearing protector according to one of claims 1 to 12, wherein the hearing protector has a mounting extension (70; 170) and the ear mould has an undercut cylindrical recess (14) open to a sound passage opening (12), for the form-fitting mounting of the mounting extension (70; 170).

14. A method for producing a hearing protector according to one of claims 1 to 13, **characterized in that** a first filter housing part (22; 122) is manufactured in an injection-moulding process in such a way that a rotationally symmetrical base part with a base-side inlet opening (26; 126) and a radial inner shoulder (36; 136) being provided behind it and an optionally double-walled circumferential wall (38; 138) of predetermined height (H38) is formed, wherein a mounting ring (40; 140) with a radially directed mounting inner shoulder (42; 142) for forming an annular support for a membrane film (30; 130) is combined with a retaining ring (30; 130) being pressed into a circular-cylindrical inner recess (46; 146) with a force fit and/or a form fit and pressing the membrane film (30; 130) flatly against the mounting inner shoulder (42; 142), preferably the retaining ring (30; 130) being made of aluminum, to form a mounting unit (60; 160).

15. The method according to claim 14, **characterized in that** the mounting ring (40; 140) with an integrated membrane film (30; 130) is pressed with a force fit and/or a form fit into the peripheral wall (38; 138) of the filter housing part (22; 122) up to an indirect or direct stop with the radial inner shoulder (36; 136).

16. The method according to claim 15, **characterized in that** a gauze net (28) inserted into the injection mould and positioned therein, which has previously been punched out of a gauze mat, is over-moulded close to the radial inner shoulder (36), and **in that** the mounting ring (40) is pressed up to a direct stop with the radial inner shoulder (36).

17. The method according to claim 15, **characterized in that** the mounting unit (160) presses a gauze net (128), which has been punched out of a gauze mat with a precise fit and placed onto the radial inner shoulder (136) preferably with circumferential guiding, against the radial inner shoulder (136) and thereby fixes said gauze net (128) in the housing (122).

18. The method in particular according to one of claims 14 to 17, **characterized in that** a cover housing part (24; 124) shutting a resonance volume (32; 132) is placed on a first filter housing part (22; 122) comprising a mounting unit (60; 160) anchored therein with a mounting ring (40; 140), a membrane film (30; 130) and a retaining ring (44; 144) with circumferential centering (54, 56; 154, 156) and is welded, preferably ultrasonically welded, on an outer circumference thereof to the first filter housing part (22; 122).

## Revendications

1. Dispositif de protection auditive passive destiné à être inséré dans le conduit auditif d'une oreille, en particulier destiné à être inséré dans un adaptateur auriculaire, avec un boîtier de filtre symétrique en rotation, composé de plusieurs parties de boîtier (22, 24 ; 122, 124), lequel loge à l'intérieur, de manière stationnaire, un filet de gaze (28 ; 128) situé en arrière d'un orifice d'entrée acoustique (26 ; 126) avec une perméabilité à l'air prédéfinie et, à une distance parallèle (AP) par rapport à celui-ci, un film de membrane (30 ; 130), en arrière duquel se trouve un volume de résonance (32 ; 132), lequel communique avec un orifice de sortie acoustique (34 ; 134), **caractérisé en ce qu'**une partie de boîtier de filtre (22 ; 122) réalise à l'arrière de l'orifice d'entrée acoustique (26 ; 126) un épaulement intérieur (36 ; 136), qui devient une section de paroi (38 ; 138) cylindrique circulaire dirigée de manière à s'éloigner de l'épaulement intérieur (36 ; 136), qui loge, pour bloquer le film de membrane (30; 130), par complémentarité de forme et/ou par frottement et/ou par liaison de matériau, une bague de montage (40 ; 140) prenant appui sur l'épaulement intérieur (36 ; 136), laquelle quant à elle forme un épaulement intérieur de montage (42 ; 142) orienté de manière radiale en tant que support annulaire pour le film de membrane (30 ; 130) bloqué par une bague de maintien (44 ; 144) enfoncée.

2. Dispositif de protection auditive selon la revendication 1, **caractérisé en ce que** la bague de montage (140) pousse le filet de gaze (128) contre l'épaulement intérieur (136) et le positionne ainsi dans le boîtier (122).

3. Dispositif de protection auditive, en particulier selon la revendication 1, avec un boîtier de filtre symétrique en rotation, composé de plusieurs parties de boîtier (22, 24), lequel loge dans son intérieur, de manière stationnaire, un filet de gaze (28) situé en arrière d'un orifice d'entrée acoustique (26), avec une perméabilité à l'air prédéfinie, et à une distance parallèle (AP) par rapport à celui-ci, un film de membrane (30), en arrière duquel se trouve un volume de résonance (32), lequel communique avec un orifice de sortie acoustique (34), **caractérisé en ce qu'**au moins la partie de boîtier de filtre (22), laquelle précisément supporte le filet de gaze (28), est réalisée en tant que partie moulée par injection en matière synthétique, et le filet de gaze (28) est enrobé côté bord et en périphérie par le boîtier de filtre (22).

4. Dispositif de protection auditive selon la revendication 3, **caractérisé en ce que** le filet de gaze (28) est tenu à distance de l'épaulement intérieur (36) d'une mesure (MA), qui est de l'ordre de grandeur de l'épaisseur (DG) du filet de gaze (28).

5. Dispositif de protection auditive selon l'une quelconque des revendications 1 à 4, **caractérisé en ce que** la bague de maintien (44; 144) est constituée d'un matériau plus dur en comparaison avec la bague de montage (40 ; 140), en particulier d'aluminium, et pousse le film de membrane (30 ; 130) à plat contre l'épaulement intérieur de montage (42 ; 142).

6. Dispositif de protection auditive selon la revendication 5, **caractérisé en ce que** la bague de montage (40 ; 140) est assemblée avec un film de membrane (30 ; 130) monté dans celle-ci par l'intermédiaire de la bague de maintien (44 ; 144) en tant qu'unité de montage (60 ; 160).

7. Dispositif de protection auditive selon l'une quelconque des revendications 1 à 6, **caractérisé en ce qu'**est réalisé de manière radiale à l'extérieur de la section de paroi (38 ; 138) cylindrique circulaire, au moins un autre volume de résonance auxiliaire (64 ; 164), lequel communique avec le volume de résonance (32 ; 132) par l'intermédiaire d'un ensemble formant ajour (62 ; 162) à configuration de section transversale prédéfinie.

8. Dispositif de protection auditive selon la revendication 7, **caractérisé en ce que** le volume de résonance auxiliaire (64 ; 164) est formé par une chambre de résonance (64 ; 164) périphérique de manière annulaire, qui communique avec le volume de résonance (32 ; 132) par l'intermédiaire d'évidements (62 ; 162) côté frontal de la section de paroi (38 ; 138) cylindrique circulaire.

9. Dispositif de protection auditive selon l'une quelconque des revendications 1 à 8, avec un boîtier de filtre symétrique en rotation, composé de plusieurs parties de boîtier (22, 24 ; 122, 124), lequel loge dans son intérieur, de manière stationnaire, un filet de gaze (28 ; 128) situé en arrière d'un orifice d'entrée acoustique (26 ; 126) avec une perméabilité à l'air prédéfinie et, à une distance parallèle (AP) par rapport à celui-ci, un film de membrane (30) ; 130, en arrière duquel se trouve un volume de résonance (32 ; 132), lequel communique avec un orifice de sortie acoustique (34 ; 134), **caractérisé en ce que** le boîtier de filtre (22, 24 ; 122, 124) est formé par deux parties de boîtier de filtre en matière synthétique (22, 24 ; 122, 124) jointes l'une à l'autre côté frontal et soudées côté extérieur, de préférence soudées par ultrasons, par l'intermédiaire d'un centrage périphérique (54, 56 ; 154, 165).

10. Dispositif de protection auditive selon la revendication 9, **caractérisé en ce que** la partie de boîtier de filtre (24 ; 124) tournée vers l'orifice de sortie acoustique (34 ; 134) est formée par un corps (24P ; 124P) en forme de plaque pouvant être soudé à l'autre partie de boîtier de filtre (22 ; 122), qui a un appendice de montage (70 ; 170) cylindrique creux contre-dépouillé pour l'ancrage amovible dans un adaptateur auriculaire (10).

11. Dispositif de protection auditive selon l'une quelconque des revendications 1 à 10, **caractérisé en ce que** le boîtier de filtre (22, 24 ; 122, 124) est constitué d'une matière synthétique thermoplastique issue du groupe des polymères synthétiques et de la famille des polyesters, de préférence d'une matière synthétique en polycarbonate (PC).

12. Dispositif de protection auditive selon l'une quelconque des revendications 1 à 11, **caractérisé en ce que** le filet de gaze (28 ; 128) et/ou le film de membrane (30 ; 130) sont produits à partir d'une matière synthétique en polyester thermoplastique, de préférence en polytéréphtalate d'éthylène (PET).

13. Adaptateur auriculaire avec un dispositif de protection auditive selon l'une quelconque des revendications 1 à 12, dans lequel le dispositif de protection auditive présente un appendice de montage (70 ; 170) et l'adaptateur auriculaire présente un creux (14) cylindrique contre-dépouillé ouvert par rapport à un orifice de passage acoustique (12) pour le logement par complémentarité de forme de l'appendice de montage (70 ; 170).

14. Procédé pour fabriquer un dispositif de protection auditive selon l'une quelconque des revendications 1 à 13, **caractérisé en ce qu'**une première partie de boîtier de filtre (22 ; 122) est fabriquée lors du procédé de moulage par injection de telle manière qu'une partie de fond symétrique en rotation apparaît avec un orifice d'entrée côté fond (26 ; 126) et un épaulement intérieur radial (36 ; 136) situé en arrière et une paroi périphérique (38 ; 138) éventuellement à double paroi d'une hauteur prédéfinie (H38), dans lequel une bague de montage (40 ; 140) est assemblée avec un épaulement intérieur de montage (42 ; 142) orienté de manière radiale pour réaliser un support annulaire pour un film de membrane (30 ; 130) avec une bague de maintien (44 ; 144) enfoncée à force et/ou par complémentarité de forme dans un creux intérieur cylindrique circulaire (46 ; 146) et poussant le film de membrane (30 ; 130) à plat contre l'épaulement intérieur de montage (42 ; 142), qui est constituée de préférence d'aluminium, en une unité de montage (60 ; 160).

15. Procédé selon la revendication 14, **caractérisé en ce que** la bague de montage (40 ; 140) avec un film de membrane (30 ; 130) intégré est pressée à force et/ou à complémentarité de forme dans la paroi périphérique (38 ; 138) de la partie de boîtier de filtre (22 ; 122) sur une butée indirecte ou directe avec l'épaulement intérieur radial (36 ; 136).

16. Procédé selon la revendication 15, **caractérisé en ce qu'**un filet de gaze (28) placé dans le moule de moulage par injection et positionné dans celui-ci, lequel a été découpé au préalable d'une natte de gaze, est enrobé à proximité de l'épaulement intérieur radial (36), et la bague de montage (40) est pressée sur une butée directe avec l'épaulement intérieur radial (36).

17. Procédé selon la revendication 15, **caractérisé en ce que** l'unité de montage (160) presse un filet de gaze (128), lequel a été découpé avec un ajustement précis d'une natte de gaze et a été placé sur l'épaulement intérieur radial (136) en étant guidé de préférence côté périphérie, contre l'épaulement intérieur radial (136) et le bloque ainsi dans le boîtier (122).

18. Procédé en particulier selon l'une quelconque des revendications 14 à 17, **caractérisé en ce qu'**une partie de boîtier formant couvercle (24 ; 124) fermant le volume de résonance (32 ; 132) est placée et est soudée, de préférence est soudée par ultrasons, côté extérieur en périphérie à la première partie de boîtier de filtre (22 ; 122), sur une première partie de boîtier de filtre (22 ; 122) avec une unité de montage (60 ; 160) ancrée dans celle-ci avec une bague de montage (40 ; 140), un film de membrane (30 ; 130) et une bague de maintien (44 ; 144) avec un centrage périphérique (54, 56 ; 154, 156).
